# EUROPEAN PATENT APPLICATION

(11) **EP 2 959 930 A1**
(43) Date of publication of application: **30.12.2015**
(21) Application number: 14174825.1
(22) Date of filing: 27.06.2014
(51) Int. Cl.: A61M 5/178, A61J 1/20, G21F 5/015

(54) **Injection assembly, method to inject a suspension, a method to swill a transporting vial, a transporting case for transporting a cartridge, a cartridge for use in the transporting case, and a transporting vial for use in the injection assembly**

(71) Applicant: QUIREM Medical BV, 7431 PH Diepenveen (NL)
(72) Inventor: Nijsen, Johannes Franciscus Wilhelmus, 7339 CL Ugchelen (NL); Van Dullemen, Marlies, 2314 EN Leiden (NL); Souhoka, Tessa, 2281 BD Rijswijk (NL); Maurice, Ingmar, 1018 AK Amsterdam (NL)
(74) Representative: De Vries & Metman

(57) **Abstract**

The present invention relates to an injection assembly for injecting a suspension, in particular a homogeneous suspension containing radioactive particles, into a medical device, such as a catheter, a syringe or a needle, the injection assembly comprising a support and a housing attached to the support. The housing is configured to hold a transporting vial containing a suspension or radioactive particles and to hold an injecting member configured to withdraw the suspension from the transporting vial and/or to inject the suspension into the medical device. The injecting member is operable from outside the housing. Further, the housing is moveable between a first condition in which the injecting member is in fluid communication to the transporting vial to withdraw the suspension from the vial, and a second condition in which the injecting member is in fluid communication to the medical device to inject the suspension into the medical device.

## Description

The present invention relates to an injection assembly for injecting a suspension, in particular a suspension containing radioactive particles, into a medical device.

The present invention further relates to a method to inject a suspension, in particular a suspension containing radioactive particles, into a medical device, a method to swill a transporting vial comprising a suspension, in particular a suspension containing radioactive particles, a transporting case for transporting a cartridge containing a transporting vial comprising a suspension, a cartridge for use in a transporting case according to the invention and a transporting vial for use in an injection assembly according to the invention.

It is known to inject a suspension containing radioactive particles into a person in order to provide a treatment to the person. In the prior art, a syringe is connected to a container, comprising at least the radioactive particles, by a tube. The syringe generates an overpressure in the container via an inlet tube by moving a fluid to the container, thereby, due to the overpressure, washing the radioactive particles out of the container via an outlet tube connected to a medical device, such as a catheter. The outlet tube comprises an additional syringe, which additional syringe is connected to a fluid source, in order to prevent radioactive particles from remaining within the outlet tube and/or the medical device.

Disadvantages of the prior art are that the injected suspension may be inhomogeneous, operating mistakes can be made, needles may be disconnected due to overpressure, leakage may occur and the outlet tube and/or the medical device may get clogged due to the inhomogeneous suspension.

The invention provides for an improved injection assembly for injecting a suspension, in particular a suspension containing radioactive particles, into a medical device, such as a catheter, a syringe or a needle.

According to an aspect of the invention an injection assembly is proposed, comprising a support and a housing attached to the support, wherein the housing is configured to hold a transporting vial, in particular within a cartridge, containing a suspension or radioactive particles and to hold an injecting member configured to withdraw the suspension from the transporting vial and/or to inject the suspension into the medical device, wherein the injecting member is operable from outside the housing. The housing is movable between a first condition in which the injecting member is in fluid communication to the transporting vial to withdraw the suspension from the vial, and a second condition in which the injecting member is in fluid communication to the medical device to inject the suspension into the medical device.

An advantage of the injection assembly is that the condition of the assembly is an indicator to an operator whether the assembly is ready to fill the injecting member with the suspension or the assembly is ready to inject the suspension into the medical device. Therefore, operating mistakes might be prevented.

A further advantage may be that as the suspension is withdrawn from the transporting vial, an underpressure is applied to the transporting vial. The risk of leakage is reduced since no forces towards the environment are applied, *e*.*g*., at connections between the elements of the injection assembly. Further, the suspension can be withdrawn from the transporting vial over a predetermined time period, such that the radioactive particles within the transporting vial are withdrawn well-spread over the time period. Thus, less radioactive particles are withdrawn from the transporting vial at once or injected into the medical device, thereby reducing the risk of clogging.

In an embodiment, the housing has an open state, in which the injecting member can be inserted into the housing, and a closed state, in which the injecting member, when present, is surrounded by the housing. As a result, an operator is prevented from accessing or touching the injecting member, which would be undesirable due to the radioactive particles within the suspension and/or exposure of the operator to radioactive radiation originating from the radioactive particles is minimized.

It is noted that the housing may be made of plumbum or a similar material, which is capable to block at least a predetermined amount of or all radioactive radiation, depending on the radioactive material used. When the injecting member is surrounded by the housing during use, such as during transport or a waiting period after filling the injecting member with the suspension, of the injection assembly, it is prevented that the operator of the injection assembly is exposed to radioactive radiation from the suspension with radioactive particles, or at least the amount of radioactive radiation outside the housing is reduced.

In one embodiment, conduits, such as tubes, are provided between the transporting vial, the injecting member and/or the medical device, wherein the conduits are interconnected by a 3-way valve, which is movable between the first condition and the second condition, preferably the 3-way valve is only moveable between the first condition and the second condition. As described above, the housing may be moveable, *e*.*g*. rotatable between a first condition and a second condition. In the first condition, the 3-way valve provides a fluid communication between the transporting vial and the injecting member, and the tube to the medical device is closed. When the housing is rotated to the second condition, the 3-way valve is operated by the housing, and thereby rotates relative to the housing and closes the tube to the transporting vial and opens the tube to the medical device. In the first condition of the housing, it is possible to withdraw the suspension or at least the radioactive particles from the transporting vial, and in the second condition, it is possible to inject the suspension into the medical device. Since it is only possible to inject the suspension into the medical device in one condition of the injection assembly, mistakes regarding injecting the suspension are prevented.

In an embodiment, one of the conduits comprises a needle configured to be inserted into the transporting vial, in particular at one end of the transporting vial, wherein an opening of the needle is inclined with respect to the flow direction therethrough, for example by bevelling the tip of the needle. The transporting vial is a closed element when delivered to a place where the transporting vial is used. In order to be able to withdraw the suspension or radioactive particles from the transporting vial, a needle is inserted into the transporting vial, which needle is connected to the injecting member by a tube or another conduit.

In an embodiment, a fluid source is connectable or connected to an end of the transporting vial, wherein the fluid source is connectable or connected to the transporting vial by means of a tube and a needle, wherein an opening of the needle is inclined with respect to the flow direction therethrough. The injecting member withdraws the suspension from the transporting vial, when the injection assembly is in the first condition. Due to the underpressure applied to the transporting vial the suspension is withdrawn from the vial and fluid is withdrawn concurrently from the fluid source into the transporting vial, leading to inwardly directed pressure applied to the connections between the different parts of the injection assembly. As a result, the risk of leakage is reduced.

Due to the bevelling of the needle tip, it is prevented that radioactive particles come to a standstill on the tip of the needle since the radioactive particles fall off the tip of the needle into the needle or back into the transporting vial and, thus, clogging at the opening of the needle is prevented.

In an embodiment, an agitating device is provided, which agitating device is configured to agitate the injecting member to keep the suspension therein homogeneous until it is injected. The agitating device may be provided within the housing. When the suspension would stand still within the injecting member, the radioactive particles in the suspension would sink to a lower part of the injecting member. It is desirable to inject the suspension with the radioactive particles as homogeneously as possible. In the context of the application, a homogeneous suspension means that the radioactive particles are as evenly distributed throughout the suspensions as possible. Agitating the injecting member, in particular when the suspension is within the injecting member, for example by moving, tilting, rotating, shaking or vibrating, contributes to distributing the radioactive particles throughout the suspension or to keep the radioactive particles distributed throughout the suspension, and thus injection of the suspension can be done in a controlled manner in which the injected volume represents a known radioactivity.

In an embodiment, at least one of the housing, the cartridge and the transporting vial is transparent, at least partly, such that the suspension is at least partly visible from the outside. The operator is therefore able to see the suspension and therewith the homogeneity of the suspension.

In an embodiment, the housing further comprises an operating member, in particular with a volume scale, for operating the injecting member. In practice, it is intended to minimize the exposure to radioactive radiation in order to avoid possible adverse effects. In this embodiment, it is possible to insert the injection member into the housing before the injection member is filled with the suspension. When the transporting vial is attached to the housing, the suspension can be withdrawn from it by the injecting member and, thereafter, be injected into the medical device by operating the operating member in the opposite direction. Exposure of the operator to radioactive radiation is minimized or even totally avoided in this manner.

Since the radioactive particles are homogeneously distributed throughout the suspension, the radioactive activity of the suspension is substantially equal at all points within the suspension. This means that, when *e.g*. the injection member is capable of containing 100 ml of the suspension and 20 ml of the suspension is injected into the medical device, 20 percent of the total radioactive activity is inserted into the medical device.

Thus the operator is able to see the amount of injected/remaining suspension and, therewith, how much radioactive activity is inserted into the medical device. An advantage of this embodiment is that the injection assembly remains simple and that it is easily determinable how much radioactive activity is injected into the medical device, i.e. into the body.

In an embodiment, the housing comprises an ejecting member configured to eject at least one of the injecting member, tubes between the transport vial and the injecting member, the transporting vial, in particular within the cartridge, and/or between the injecting member and the medical device. Since the injection assembly is applied with radioactive particles, it is possible that any radioactive material is left behind in a part of the injection assembly when the suspension is injected into the medical device. In order to minimize exposure of the operator to radioactive radiation, it is advantageous that the elements of the injection assembly, such as the injecting member, the tubes and the 3-way valve, may be ejected without touching the parts or articles.

According to an aspect of the invention, a method to inject a suspension, in particular a suspension containing radioactive particles, into a medical device, such as a catheter or a needle, is proposed, comprising the steps of:
inserting a transporting vial into the housing of the injection assembly; connecting the transporting vial to an injecting member of the injection assembly; placing the housing in the first condition such that the vial is in fluid communication to the injecting member; withdrawing a suspension from the transporting vial by means of the injecting member and/or into the injecting member, moving the housing of the injection assembly from the first condition to the second condition such that the injecting member is in fluid communication to the medical device, and injecting the withdrawn suspension into the medical device.

The suspension may be agitated to keep it homogeneous until it is injected, in particular the suspension may be rotated to keep it homogeneous until it is injected.

In an embodiment, the step of injecting the withdrawn suspension into the medical device comprises the steps of: injecting a predetermined amount of suspension into the medical device; displacing the medical device to a predetermined position; and injecting a predetermined amount of suspension into the medical device at the predetermined position, wherein the steps of displacing the medical device and injecting a predetermined amount of suspension into the medical device might be repeated.

According to another aspect of the invention, a method to swill a transporting vial comprising a suspension, in particular a suspension containing radioactive particles, is proposed, comprising the steps of connecting the bottom of the transporting vial to an outlet tube, such that the inlet opening of the outlet tube is spaced from the bottom of the transporting vial, connecting the upper end of the transporting vial to a fluid source comprising a fluid, such that the fluid source and the transporting vial are in fluid communication, and supplying fluid from the fluid source to the transporting vial and creating an underpressure in the outlet tube to as to create a swirl in the fluid from the bottom of the transporting vial towards and into the outlet tube, thereby swilling the complete suspension from the transporting vial.

An advantage of this method is that all radioactive particles contained within the transporting vial may be swilled out of the transporting vial. It is, therefore, known how many radioactive particles are swilled out of the transporting vial and into *e*.*g*. the injecting member. The doses of the injected particles is therewith controllable.

A further advantage is that substantially no radioactive particles are left behind in the transporting vial after the latter is swilled. Therefore, the radioactivity of the suspension within the injecting member is known, since the radioactivity is substantially the same as the radioactivity of the suspension within the transporting vial.

According to an aspect of the invention a transporting case for transporting a cartridge containing a vial comprising a suspension, in particular a suspension with at least radioactive particles, is proposed, the transporting case comprising a base member and a lid placeable on the base member. The base member comprises a bottom part and a holding part configured to hold the cartridge at least partly, and
wherein the lid surrounds the holding part at least partly, and the lid comprises a retaining part configured to retain a part of the cartridge temporarily.

An advantage of the transporting case is that the cartridge can be placed around or can be removed from the transporting vial without an operator touching at least one of the transporting vial and the cartridge.

In an embodiment the lid comprises a closable opening, such that the transporting vial within the transporting case is accessible from the outside thereof, *e*.*g*. with an injection needle to insert a fluid into the transporting vial.

In an embodiment, the transporting case is configured to block γ-radiation (Gamma-radiation) originating from the suspension within the transporting case. The blocking function can be achieved by adjusting the wall thickness of the transporting case or by choosing a suitable material. In practice, plumbum is often used for producing such objects.

According to an aspect of the invention a cartridge for use in a transporting case according to the invention is proposed, the cartridge comprising a receiving part adapted to receive the vial containing a suspension, in particular a suspension with radioactive particles, and a lid adapted to close off the receiving part, when the vial is received in the receiving part.

In an embodiment, the cartridge is configured to block at least one of α- and β-radiation (Alpha- and Beta-radiation) originating from the suspension within the transporting vial placed in the cartridge.

In an embodiment, the cartridge comprises a septum at least at one of the top side and bottom side of the cartridge to provide access to the transporting vial within the cartridge.

According to an aspect of the invention a vial for use in an injection assembly according to the invention is proposed.

Aspects of the invention will be explained in greater detail by reference to exemplary embodiments of the invention shown in the drawings, in which:
Figs. 1-3 illustrate the steps of preparing a suspension by means of a transporting vial, a cartridge and a transporting case, shown in side view;
Figs. 4-6 are perspective views of the injection assembly in different conditions thereof; and
Fig. 7 is a cross-section of the transporting vial when attached to the injection assembly.

It should be appreciated, however, that these embodiments may not be construed as limiting the scope of protection for the present invention.

In nuclear medicine, the injection of a radioactive material is, *i.a*., applied for the treatment of one or more tumours. The radioactive material can be injected locally, such that the only a specific part of the body of a patient receives the radioactive radiation, thereby saving as much healthy tissue as possible. It is further desirable to minimize the exposure of a person to radioactive radiation.

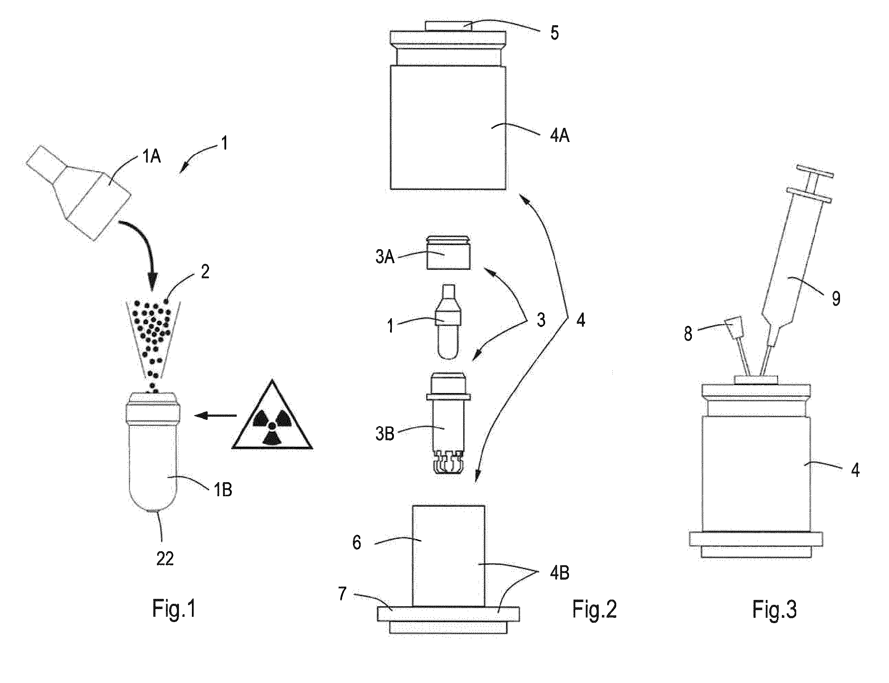

As shown in Fig. 1, a transporting vial 1 is provided which can be filled with a material 2 that may comprise a plurality of microspheres. In this embodiment, the microspheres are made of holmium. The transporting vial 1 comprises two parts 1A, 1B, such that the material 2 can be inserted easily and thereafter the transporting vial 1 is closed.

It is noted that at the time of filling the transporting vial 1 with the material 2, the material 2 is not necessarily emitting radioactive radiation. In the embodiment shown in Fig. 1, the material 2 inserted into the transporting vial 1 is not emitting radioactive radiation at the time of insertion. When the material 2 is inserted into the transporting vial 1, the material 2 is radiated, which leads to a radioactive material 2 within the transporting vial 1. The transporting vial 1 has a part 22 with a thinner wall in comparison with the remaining wall of the transporting vial 1, such that *e.g*. a needle may be inserted into the transporting vial 1 from below.

It is noted that the material 2 may be radiated in a transporting vial 1 and thereafter may be transferred to another transporting vial 1.

Fig. 2 shows a transporting case 4, cartridge 3, and the transporting vial 1. A lid 4A of the transporting case 4 comprises a retaining part (not shown) to retain a cartridge lid 3A of the cartridge 3 at least temporarily. A release button 5 is provided with the lid 4A of the transporting case, in order to be able to release the cartridge lid 3A of the cartridge 3 from the lid 4A of the transporting case 4.

In this embodiment, the cartridge lid 3A of the cartridge 3 may be attached to the lid 4A of the transporting case 4 before it is attached to a receiving part 3B of the cartridge 3. Thus, the lid 4A of the transporting case 4 holds the cartridge lid 3A of the cartridge and the lid 4A may be used to attach the cartridge lid 3A to the receiving part 3B of the cartridge 3.

The transporting case 4 comprises a base member 4B comprising a bottom part 7 and a holding part 6. In practice, the receiving part 3B of the cartridge 3 is placed in the holding part 6 of the base member 4B of the transporting case 4 and accordingly the transporting vial 1 is placed in the receiving part 3B of the cartridge 3. The lid 4A of the transporting case 4 holding the cartridge lid 3A of the cartridge 3 is placed over the holding part 6 of the base member 4B of the transporting case 4, while the receiving part 3B of the cartridge 3 and the transporting vial 1 are positioned within the holding part 6. The cartridge lid 3A and the receiving part 3B of the cartridge 3 can be connected to each other, such that the lid 4A of the transporting case 4 can be lifted while holding the cartridge 3 comprising the transporting vial 1. The diameter of the bottom part 7 is larger than that of the holding part 6. and is adapted to both support lid 4A and allows the transporting case 4 to be placed on a surface (not shown) in a robust and reliable manner.

The cartridge lid 3A and the receiving part 3B may both be provided with a septum (not shown), such that it is possible to gain access to the transporting vial 1 for example by means of a needle without causing the risk of leakage.

It is noted that the cartridge 3 is made of a material capable to block β-radiation (Beta-radiation) and that the transporting case 4 is made of a material capable to block γ-radiation (Gamma-radiation). The transporting case 4 can be made of plumbum or any other suitable material. It is advantageous that β- and γ-radiation is blocked by the cartridge 3 and the transporting case 4 since a person carrying the transporting case 4 is not exposed to the radioactive radiation.

Fig. 3 shows an optional step of preparing the suspension containing radioactive particles 2. In the state as shown in Fig. 3, the transporting vial 1 containing the radioactive material 2 is placed in the transporting case 4 together with the cartridge 3. The transporting vial 1 only contains the radioactive material 2. In an embodiment it may be desirable to already add a fluid to the radioactive material 2 in order to realise a suspension.

Fig. 3 shows how a fluid, also called a buffer solution, is supplied to the transporting vial 1. In this embodiment, the lid 4A of the transporting case 4 comprises a removable part (not shown) to provide access to the septum of the cartridge lid 3A of the cartridge 3 and the transporting vial 1 within the transporting case 4. A needle 8 comprising a ventilation filter (not shown) is inserted into the transporting vial 1 through the septum of the cartridge lid 3A of the cartridge 3. Subsequently, a syringe 9 is filled with the buffer solution, which may be water, a saline solution or any other suitable fluid. The syringe 9 is inserted into the transporting vial 1 through the lid 4A of the transporting case 4 and the septum of the cartridge lid 3A of the cartridge 3. Thereafter, the fluid in the syringe 9 is injected into the transporting vial 1 and concurrently air escapes from the transporting vial 1 through the needle 8. After injecting the fluid into the transporting vial 1, the needle 8 and the syringe 9 are removed and the suspension is ready to be transferred to the injection assembly, before being injected into a person.

It is noted that the suspension also can be prepared when the transporting vial 1 is connected to the injection assembly 10 as explained below.

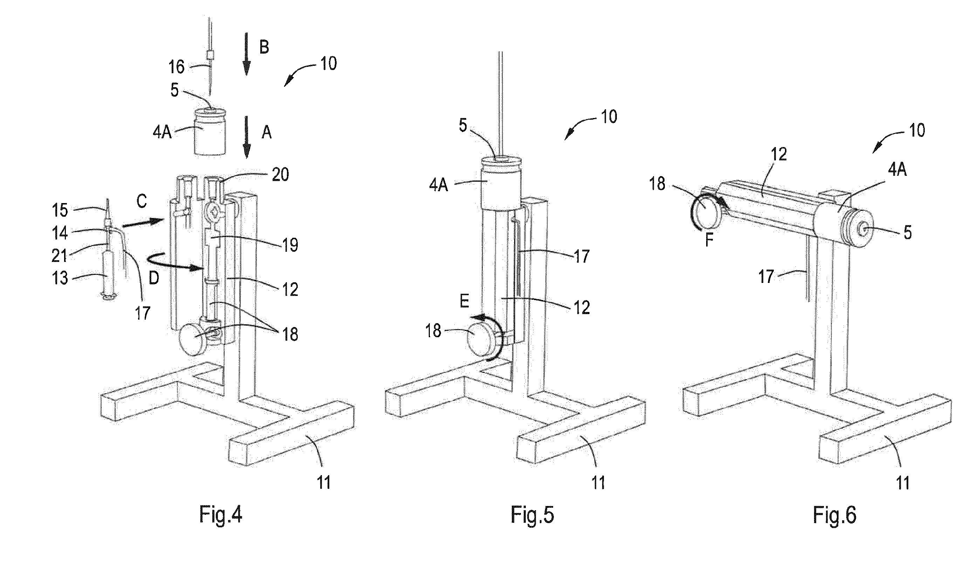

Figs. 4-6 show different conditions, such as orientations and states of the injection assembly 10 according to the invention. The injection assembly 10 comprises a support 11 to place the injection assembly 10 on a base, such as a table (not shown). A housing 12 is rotatably attached to the support 11, such that it can rotate about a substantially horizontal axis between a first, in this case upright orientation, as shown in Figs. 4 and 5, and a second, more horizontal orientation, as shown in Fig. 6. The housing 12 has an open state as shown in Fig. 4 and a closed state as shown in Figs. 5 and 6.

In Fig. 4, it is shown that the housing 12 is in the open state, such that it is possible to introduce the disposable part of the injection assembly, i.a. comprising the injecting member 12, into the housing 12 comprising a recess 19 configured to hold the injecting member 13. In this embodiment, the injecting member 13 is configured as a syringe. An opening of the injecting member 13 is connected to a conduit such as a tube 21 comprising a 3-way valve 14, and a needle 15 and tube 17 connected to the tube 21 through the 3-way valve 14. The needle 15 is intended to be inserted into the transporting vial 1 through a bottom part 22 thereof, when attached to the injection assembly 10. The 3-way valve 14 is rotatable relative to the housing between a first orientation, in which the tube 21 of the injecting member 13 is in fluid communication with the needle 15 and thus the transporting vial 1 to withdraw the suspension from the transporting vial 1, and a second orientation, in which the injecting member 13 is in fluid communication to the medical device through tube 17.

In an embodiment, the 3-way valve 14 is only rotatable between the first orientation and the second orientation.

In practice, the injecting member 13 is introduced into the housing 12, together with the tubes 17, 21, the 3-way valve and the needle 15, as indicated with arrow C, during which at least a part of the injecting member 13 is (automatically) connected to an operating member 18, such that the injecting member 13 is operable from outside the housing 12. Subsequently, the housing 12 is closed as indicated by arrow D, such that the injecting member 13 is surrounded by the housing 12 during use.

In an embodiment, the housing 12 is made of plumbum or any other suitable material such that radioactive radiation originating from the suspension within the housing 12 cannot reach an operator operating the injection assembly 10.

When the housing 12 is in the closed state, it is possible to place the lid 4A of the transporting case 4, together with the cartridge 3 and the transporting vial 1 over a part 20 of the housing 12 as indicated with arrow A, which part 20 is open at the top and hollow so as to be able to hold the transporting vial 1. At the same time, the needle 15 is introduced into the transporting vial 1 through the septum (not shown) at the bottom of the receiving part 3B of the cartridge. This situation is shown in Fig. 5.

In a particular embodiment, a needle 16 is introduced into the transporting vial 1 at the top side thereof as indicated with arrow B, which needle 16 is connected to a fluid source (not shown). The fluid source may comprise water, a saline solution or any other suitable fluid.

When the injecting member 13 is connected to the transporting vial 1 and the injection assembly 10 is in the first orientation, the operating member 18 may be rotated as indicated with arrow E. During rotation of the operating member, the injecting member 13 applies an underpressure to the transporting vial 1 by for example withdrawing a plunger manually or by a motor and, optionally, fluid from the fluid source (not shown) is supplied to the transporting vial 1 to as to create a swirl in the suspension of the transporting vial 1 towards and into the needle 15, thereby swilling the complete suspension from the transporting vial 1 and generating a relatively homogeneous suspension within the injecting member 13.

In an embodiment, each of the tubes 16, 17 may be provided with an one-way valve (not shown) in order to prevent the suspension from flowing back to the transporting vial 1, when in the first orientation, and to prevent the suspension from flowing back to the injecting member 13, when in the second orientation. In this manner, a risk to operation failures is reduced.

Subsequently, the housing 12 is rotated to the second orientation as indicated in Fig. 6. During rotation of the housing 12, the 3-way valve 14 also rotates relative to the housing 12 from the first orientation, in which the injecting member 13 is in fluid communication to the transporting vial 1, to the second orientation, in which the injecting member 13 is in fluid communication to the medical device (not shown) indicated with tube 17.

The housing 12 may be configured to rotate and/or tilt or otherwise shake, vibrate or move the injecting member 13 during presence of the suspension within the injecting member 13, in order to agitate the suspension and thereby to keep it homogeneously. An agitating device, such as two or more rollers (not shown) may be provided to move the injecting member 13. The injecting member 13 may be placed on the rollers, *e.g*. when the housing 12 is in the second orientation, and the rollers may be configured to rotate the injecting member 13 about a longitudinal axis of the injecting member 13. The rollers may rotate the injecting member 13 completely about the longitudinal axis, and/or may rotate the injecting member 13 reciprocally. The injecting member 13 may be rotated over 180°, in particular reciprocally. In an embodiment, the injecting member 13 is rotated reciprocally over 180° four times within approximately 5 seconds. As a result, the suspension within the injecting member 13 is kept homogeneously. The injecting member 13 may be rotated continuously or intermittently.

In a further embodiment, the injecting member 13 is shaken in vertical direction, *e*.*g*. when the housing 12 and the injecting member 13 are in the second orientation, in a direction transversal to the longitudinal axis of the injecting member 13. The injecting member 13 may be shaken continuously or for a predetermined time period, for instance 10 seconds. After shaking the injecting member 13 for the predetermined time period, the injecting member 13 may stand still for a predetermined time period, for instance 5 seconds, and subsequently the injecting member 13 may be shaken again.

It is also possible that the injecting member 13 is rotated eccentrically about the longitudinal axis, for instance by means of a vortex device (not shown).

The housing comprises a transparent part (not shown) and the injecting member 13 is at least partly transparent, such that the homogeneity of the suspension in the injecting member is at least partly visible from the outside, and at least one of an additional operating member 23 and the operating member 18 is provided with a volume scale (not shown). Since the suspension within the injecting member 13 is homogeneous and the suspension comprises a certain amount of radioactive material representing a total radioactive dose, an injected percentage of the total suspension represents the same injected percentage of the radioactive dose. Thus, when the operator is able to determine the injected and/or remaining volume of the suspension, the operator is able to calculate the injected radioactive dose.

In the second orientation of the housing 12, the operating member 18 is rotated in the direction indicated with arrow F, which is opposite to the direction indicated with arrow E, thereby injecting the suspension into the medical device (not shown) via the tube 17. It is noted that the suspension is injected at once or is divided over multiple doses, such that it is possible to inject the suspension at different places of for example an organ of a patient. This gives the opportunity to treat several tumours in one organ individually.

It is noted that in a particular embodiment, the transporting case, needs to be removed from the housing before it is possible to open the housing of the injection assembly and ejecting the parts, such as the injecting member, the tubes and the 3-way valve.

In the embodiment shown in Figs. 4-6, the housing 12 may be opened only when the lid 4A of the transporting case 4 is not attached to the injection assembly. When the lid 4A of the transporting case 4 is removed from the housing 12, the housing is opened and an ejecting member (not shown) can be activated, thereby ejecting at least one of the injecting member 13, the tubes 17, 21, the transporting vial 1 together with the cartridge 3 and the needle 15. In case the ejecting member is activated when a bin is positioned below the housing, there is no need to touch the ejected articles.

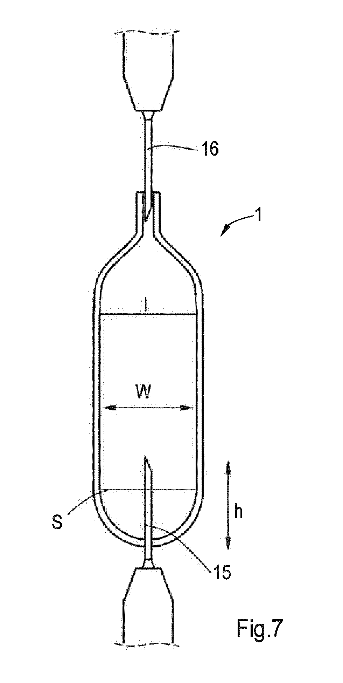

Fig. 7 shows a cross-section of the transporting vial 1 when attached to the injection assembly 10 before withdrawing the suspension from the transporting vial 1. As can be seen, the needle 15 is introduced into the transporting vial 1 through the bottom thereof. The height h of the needle within the transporting vial 1 is larger than the level s of the initial amount of settled down material 2, such as holmium, within the transporting vial 1. The transporting vial 1 has a predetermined diameter W, which contributes to the generation of a swirl within the transporting vial 1. In this embodiment, the suspension or at least the fluid thereof, has an initial level I as shown in the figure. It is, however, also possible that the transporting vial 1 only comprises the radioactive material 2 and that the fluid is added at a later stage, for example via the needle 16.

In the embodiment shown in Fig. 7 the inlet opening of the needle 15, which forms the outlet tube, is inclined with respect to the flow direction therethrough to create a bevelled tip. It is therewith prevented that radioactive particles come to a standstill on the edge of the inlet opening of the needle 15. It also promotes the swirl in the fluid as the fluid is sucked-in from the side. The outlet opening of the needle 16, which forms the inlet tube, is also inclined or bevelled with respect to the flow direction therethrough, such that the flow of the fluid is directed to the wall of the transporting vial 1. As a result, the fluid flows along the wall, thereby generating a swirl at the bottom of the transporting vial 1 towards and into the needle 15, thereby swilling the complete suspension from the transporting vial 1. As a result, all radioactive particles 2 are swilled out of the transporting vial 1.

It is noted that the drawings are schematic, not necessarily to scale and that details that are not required for understanding the present invention may have been omitted. The terms "upward", "downward", "below", "above", and the like relate to the embodiments as oriented in the drawings, unless otherwise specified. Further, elements that are at least substantially identical or that perform an at least substantially identical function are denoted by the same numeral.

The invention is not restricted to the above-described embodiments, which can be varied in a number of ways within the scope of the claims. For example, moving the housing between the first and second condition could also include shifting between two positions. It is also possible that the suspension is injected into the medical device and therewith into, *e.g*., a liver of a patient using an image-guiding system, such as MRI, SPECT, PET or any other nuclear imaging device.

In the case of MRI and holmium, the obtained images are distorted by the holmium microspheres. The distortion results in blackening of the images. By determining the degree of blackening at a certain position of an image, it is possible to determine the amount of microspheres present at that certain position of the image. The degree of blackening is linear with the amount of microspheres. It is thus possible to determine the distribution of the microspheres throughout the organ and/or body of a person in which the microspheres are injected. Further, it is possible to determine the dose distribution throughout an organ, such as the liver, and based on that determination it can be decided whether or not to inject more microspheres into a certain part of the liver.

It is described that the suspension is injected into a medical device. It is however also possible that the injection assembly is used for distributing the suspension over a plurality of transporting vials, also called personal dose vials, which are subsequently used for injecting the suspension into various patients. In this case, the initial transporting vial comprises more suspension than in case the suspension is injected into a medical device and the initial transporting vial is called a suspension preparation vial. A device can be provided to connect a plurality of transporting vials to the initial transporting vial one by one. It is possible that an additional syringe is provided, which additional syringe is connected or connectable to a fluid source and/or an outlet tube and/or a medical device, to swill the outlet tube and/or the medical device, in order to prevent radioactive particles from remaining within the outlet tube and/or the medical device.

## Claims

1. Injection assembly for injecting a suspension, in particular a suspension containing radioactive particles, into a medical device, such as a catheter, a syringe or a needle, the injection assembly comprising:
a support; and
a housing attached to the support, wherein the housing is configured to hold a transporting vial, in particular within a cartridge, containing a suspension or radioactive particles and to hold an injecting member configured to withdraw the suspension from the transporting vial and/or to inject the suspension into the medical device,
wherein the injecting member is operable from outside the housing, and
wherein the housing is movable between a first condition in which the injecting member is in fluid communication to the transporting vial to withdraw the suspension from the transporting vial, and a second condition in which the injecting member is in fluid communication to the medical device to inject the suspension into the medical device.

2. Injection assembly according to claim 1, wherein conduits are provided between the transporting vial, the injecting member and/or the medical device, wherein the conduits are interconnected by a 3-way valve, which is movable between the first condition and the second condition, preferably wherein the 3-way valve is only moveable between the first condition and the second condition.

3. Injection assembly according to claim 2, wherein one of the conduits comprises a needle configured to be inserted into the transporting vial, in particular at one end of the transporting vial, wherein an opening of the needle is inclined with respect to the flow direction therethrough.

4. Injection assembly according to any one of the preceding claims, wherein a fluid source is connectable or connected to an end of the transporting vial, wherein the fluid source is connectable or connected to the transporting vial by means of a conduit and a needle, wherein an opening of the needle is inclined with respect to the flow direction therethrough.

5. Injection assembly according to any one of the preceding claims, wherein an agitating device is provided, which agitating device is configured to agitate, in particular to rotate, the injecting member to keep the suspension therein homogeneous until it is injected.

6. Injection assembly according to any one of the preceding claims, wherein at least one of the housing, the cartridge and the transparent is transparent, at least partly, such that the suspension is at least partly visible from the outside.

7. Injection assembly according to any one of the preceding claims, wherein the housing further comprises an operating member, in particular with a volume scale, for operating the injecting member.

8. Injection assembly according to any one of the preceding claims, further comprising an ejecting member configured to eject at least one of the injecting member, tubes between the transport vial and the injecting member, the transporting vial, in particular within the cartridge, and/or between the injecting member and the medical device.

9. Method to inject a suspension, in particular a suspension containing radioactive particles, into a medical device, such as a catheter, syringe or a needle, the method comprising the steps of:
inserting a transporting vial into a housing of an injection assembly;
connecting the transporting vial to an injecting member of the injection assembly;
placing the housing in a first condition such that the transporting vial is in fluid communication with the injecting member;
withdrawing a suspension from the transporting vial by means of the injecting member and/or into the injecting member,
moving the housing of the injection assembly from the first condition to a second condition such that the injecting member is in fluid communication with the medical device, and
injecting the withdrawn suspension into the medical device.

10. Method according to claim 9, wherein the suspension is agitated to keep it homogeneous until it is injected, in particular wherein the suspension is rotated to keep it homogeneous until it is injected.

11. Method according to claim 10 or 11, wherein the step of injecting the withdrawn suspension into the medical device comprises the steps of: injecting a predetermined amount of suspension into the medical device; displacing the medical device to a predetermined position; and injecting a predetermined amount of suspension into the medical device at the predetermined position, wherein the steps of displacing the medical device and injecting a predetermined amount of suspension into the medical device might be repeated.

12. Method to swill a transporting vial comprising a suspension, in particular a suspension containing radioactive particles, the method comprising the steps of:
connecting the bottom of the transporting vial to an outlet tube, such that the inlet opening of the outlet tube is spaced from the bottom of the transporting vial,
connecting the upper end of the transporting vial to a fluid source comprising a fluid, such that the fluid source and the transporting vial are in fluid communication, and
supplying fluid from the fluid source to the transporting vial and creating an underpressure in the outlet tube to as to create a swirl in the fluid from the bottom of the transporting vial towards and into the outlet tube, thereby swilling the complete suspension from the transporting vial.

13. Transporting case for transporting a cartridge containing a transporting vial comprising a suspension, in particular a suspension with at least radioactive particles, the transporting case comprising:
a base member; and
a lid placeable on the base member,
wherein the basis member comprises a bottom part and a holding part configured to hold the cartridge at least partly, and
wherein the lid surrounds the holding part at least partly, and the lid comprises a retaining part configured to retain a part of the cartridge temporarily.

14. Cartridge for use in a transporting case according to claim 13, the cartridge comprising:
a receiving part adapted to receive a transporting vial containing a suspension, in particular a suspension with radioactive particles; and
a lid adapted to close off the receiving part, when the transporting vial is received in the receiving part.

15. Transporting vial for use in an injection assembly according to any one of the claims 1-8 or in a method according to any one of the claims 9-12.
